# EUROPÄISCHE PATENTANMELDUNG

(11) **EP 3 692 942 A1**
(43) Veröffentlichungstag der Anmeldung: **12.08.2020**
(21) Anmeldenummer: 19155676.0
(22) Anmeldetag: 06.02.2019
(51) Int. Cl.: A61C 1/00, A61B 17/00

(54) **STEUERMODUL ZUR STEUERUNG EINES VOLUMENSTROMS EINES GASFÖRMIGEN ANTRIEBSMEDIUMS EINES DENTALEN ODER MEDIZINISCHEN BEHANDLUNGSWERKZEUGS**

(71) Anmelder: W & H Dentalwerk Bürmoos GmbH, 5111 Bürmoos (AT)
(72) Erfinder: RUSCH, Stefan, 5120 St. Pantaleon (AT); REITER, Michael, Dr., 5061 Elsbethen (AT)
(74) Vertreter: Benda, Ralf

(57) **Zusammenfassung**

Steuermodul (10; 10A) zur Steuerung eines Volumenstroms eines gasförmigen Antriebsmediums eines pneumatisch angetriebenen Behandlungswerkzeugs (5), wobei das gasförmige Antriebsmedium durch eine Schlauchleitung (4; 15) zu dem pneumatisch angetriebenen Behandlungswerkzeug (5) fließt und wobei an der Schlauchleitung (4; 15) ein Stellelement (9) vorgesehen ist, das den Volumenstrom des durch die Schlauchleitung (4; 15) fließenden gasförmigen Antriebsmediums stellt, um damit die Drehzahl und/ oder Leistung des pneumatisch angetriebenen Behandlungswerkzeugs (5) zu steuern, wobei das Steuermodul (10; 10A) das Stellelement (9) und einen Funkempfänger (8) aufweist, der ausgebildet ist, ein drahtlos übertragenes Funk-Stellsignal einer Funk-Fußsteuerung (3) zu empfangen, wobei der Funkempfänger (8) und das Stellelement (9) kommunikativ verbunden sind, so dass das Stellelement (9) auf Basis des drahtlos übertragenen und empfangenen Funk-Stellsignals den Volumenstrom des durch die Schlauchleitung (4; 15) fließenden gasförmigen Antriebsmediums stellt.

## Beschreibung

Die vorliegende Erfindung betrifft ein Steuermodul zur Steuerung eines Volumenstroms eines gasförmigen Antriebsmediums eines dentalen oder medizinischen, pneumatisch angetriebenen Behandlungswerkzeugs.

In dem Patent US 5,107,899 ist insbesondere in den Figuren 1A und 2 die übliche Versorgung eines dentalen oder medizinischen, pneumatisch angetriebenen Behandlungswerkzeugs dargestellt: von einer Druckluftquelle, d.h. einem Kompressor, wird einer dentalen oder medizinischen Behandlungseinheit, mit der das pneumatisch angetriebenen Behandlungswerkzeug verbunden ist, Druckluft zugeführt und über eine Schlauchleitung an eine Fußsteuerung weitergeleitet. Der Anwender betätigt über ein Pedal der Fußsteuerung ein Ventil in der Fußsteuerung, das den Volumenstrom der Druckluft in der Schlauchleitung stellt. Die Druckluft fließt anschließend zurück durch die Behandlungseinheit und weiter zu dem pneumatisch angetriebenen Behandlungswerkzeug, wobei in Abhängigkeit des durch den Anwender gestellten Volumenstroms der Druckluft die Drehzahl oder Leistung des pneumatisch angetriebenen Behandlungswerkzeugs steuerbar ist.

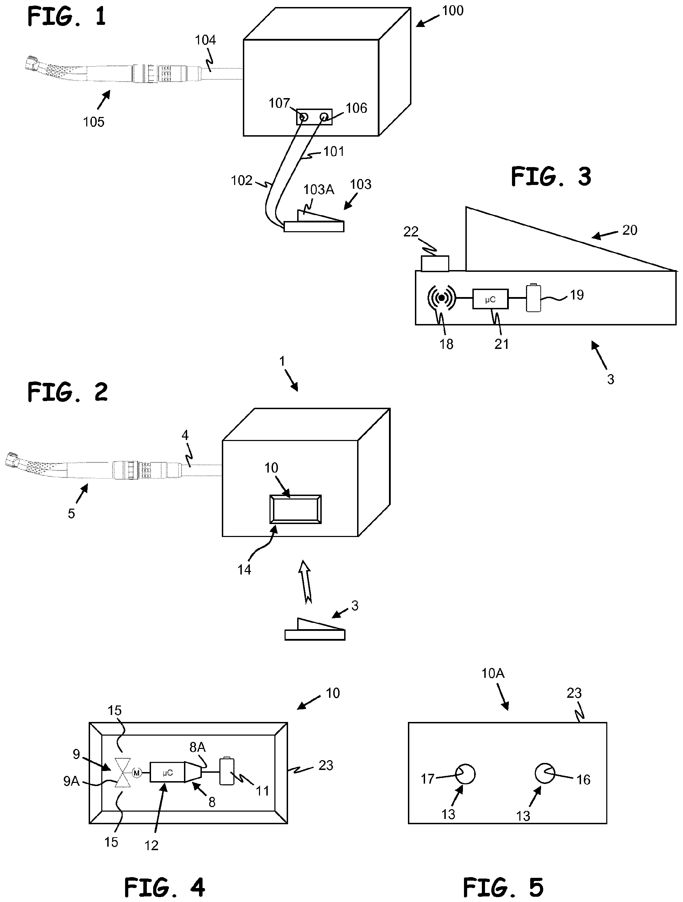

Die Figur 1 zeigt in einer vereinfachten, schematischen Ansicht die technische Ausgestaltung wie sie in dem Patent US 5,107,899 offenbart ist: eine dentale oder medizinische, insbesondere pneumatisch betriebene Behandlungseinheit 100 ist über eine erste Schlauchleitung 101 und eine zweite Schlauchleitung 102 mit einer pneumatischen Fußsteuerung 103 verbunden. Durch eine Ausgangsöffnung 106 und die erste Schlauchleitung 101 gelangt Druckluft von einer Druckluftquelle (nicht dargestellt) zu der Fußsteuerung 103, wobei über ein Pedal 103A der Volumenstrom der Druckluft stellbar ist. Über die zweite Schlauchleitung 102 und eine Einlassöffnung 107 gelangt der gestellte Volumenstrom der Druckluft zurück in die Behandlungseinheit 100 und wird von dort über eine Schlauchleitung 104 einem dentalen oder medizinischen, pneumatisch angetriebenen Behandlungswerkzeug 105 zugeführt, wobei die Druckluft eine Antriebsvorrichtung des Behandlungswerkzeugs 105 antreibt, insbesondere in Rotation versetzt. Durch das Stellen der Druckluft ist somit die Drehzahl oder Leistung des dentalen oder medizinischen, pneumatisch angetriebenen Behandlungswerkzeugs 105 durch den Anwender steuerbar.

Ein Nachteil bei diesen bekannten dentalen oder medizinischen Behandlungseinheiten sind die Schlauchleitungen zwischen dem pneumatisch angetriebenen Behandlungswerkzeug bzw. der dentalen oder medizinischen Behandlungseinheit und der Fußsteuerung. Die Schlauchleitungen begrenzen die Möglichkeiten der Positionierung der Fußsteuerung und stellen eine Stolpergefahr dar.

Es wäre daher wünschenswert eine Möglichkeit zu schaffen, die Drehzahl oder Leistung eines dentalen oder medizinischen, pneumatisch angetriebenen Behandlungswerkzeugs durch eine schlauchlose und drahtlose Fußsteuerung zu steuern.

Der vorliegenden Erfindung liegt daher die Aufgabe zugrunde, dem Anwender eine einfache und kostengünstige Möglichkeit zu bieten, die Drehzahl oder Leistung eines dentalen oder medizinischen, pneumatisch angetriebenen Behandlungswerkzeugs mit einer schlauchlosen und drahtlosen Fußsteuerung zu steuern. Insbesondere soll der Anwender für die schlauchlose und drahtlose Steuerung sein vorhandenes pneumatisch angetriebenes Behandlungswerkzeug und seine vorhandene dentale oder medizinische, pneumatische Behandlungseinheit weiterverwenden können, so dass die Investitionskosten für die schlauchlose und drahtlose Steuerung möglichst gering sind.

Diese Aufgabe wird gemäß der vorliegenden Erfindung durch ein Steuermodul zur Steuerung eines Volumenstroms eines gasförmigen Antriebsmediums eines dentalen oder medizinischen, pneumatisch angetriebenen Behandlungswerkzeugs mit den Merkmalen des Anspruchs 1, durch ein Set mit einem derartigen Steuermodul und durch eine dentale oder medizinische Behandlungseinheit mit einem derartigen Steuermodul und durch ein Verfahren nach Anspruch 15 zum Umrüsten oder Nachrüsten einer dentalen oder medizinischen Behandlungseinheit gelöst. Besonders vorteilhafte Ausführungsformen sind in den Unteransprüchen angeführt.

Es ist ein Steuermodul zur (drahtlos und/ oder schlauchlos und/ oder funkgesteuert) Steuerung eines Volumenstroms eines gasförmigen Antriebsmediums eines dentalen oder medizinischen, pneumatisch angetriebenen Behandlungswerkzeugs vorgesehen, wobei das gasförmige Antriebsmedium durch eine Schlauchleitung zu dem pneumatisch angetriebenen Behandlungswerkzeug fließt und wobei an der Schlauchleitung ein Stellelement vorgesehen ist, das ausgebildet ist, den Volumenstrom des durch die Schlauchleitung fließenden gasförmigen Antriebsmediums zu stellen, um damit die Drehzahl und/ oder Leistung des dentalen oder medizinischen, pneumatisch angetriebenen Behandlungswerkzeugs zu steuern. Das Steuermodul umfasst das Stellelement und einen Funkempfänger, der ausgebildet ist, ein drahtlos übertragenes Funk-Stellsignal einer Funk-Fußsteuerung zu empfangen, wobei der Funkempfänger und das Stellelement kommunikativ verbunden sind, so dass das Stellelement auf Basis des drahtlos übertragenen und empfangenen Funk-Stellsignals den Volumenstrom des (durch die Schlauchleitung) zu dem dentalen oder medizinischen, pneumatisch angetriebenen Behandlungswerkzeugs fließenden gasförmigen Antriebsmediums stellt.

Mithilfe des Steuermoduls ist es somit in vorteilhafter Weise möglich, den Volumenstrom eines gasförmigen Antriebsmediums, insbesondere von Druckluft eines dentalen oder medizinischen, pneumatisch angetriebenen Behandlungswerkzeugs mit einer Funk-Fußsteuerung drahtlos und/ oder schlauchlos und/ oder funkgesteuert zu steuern. Durch die Steuerung des Volumenstroms ist die Drehzahl und/ oder Leistung des dentalen oder medizinischen, pneumatisch angetriebenen Behandlungswerkzeugs und/ oder einer Werkzeughalterung des pneumatisch angetriebenen Behandlungswerkzeugs und/ oder einer Antriebsvorrichtung des Behandlungswerkzeugs, insbesondere einer rotierenden Antriebsvorrichtung veränderbar.

Gemäß einem im Folgenden noch detaillierter beschriebenen Ausführungsbeispiel ist das Steuermodul vorzugsweise als Nachrüst-Steuermodul oder Umrüst-Steuermodul ausgebildet, wodurch in vorteilhafter Weise die Weiterverwendung des vorhandenen pneumatisch angetriebenen Behandlungswerkzeugs und der vorhandenen dentalen oder medizinischen, insbesondere rein pneumatischen Behandlungseinheit möglich sind.

Das Steuermodul ist vorzugsweise als separates Modul ausgebildet, insbesondere wenn das Steuermodul als Nachrüst-Steuermodul oder Umrüst-Steuermodul ausgebildet ist, das mit der dentalen oder medizinischen Behandlungseinheit, insbesondere deren Ausgangsöffnung und Einlassöffnung und/ oder der Schlauchleitung für das gasförmige Antriebsmedium, verbindbar ist (gegebenenfalls nach Entfernen der Schlauchleitungen, welche die dentale oder medizinische Behandlungseinheit mit der Fußsteuerung verbinden). Das separate Steuermodul ist zum Beispiel als Steckmodul ausgebildet, das an die dentale oder medizinische Behandlungseinheit, insbesondere deren Ausgangsöffnung und Einlassöffnung ansteckbar ist. Selbstverständlich sind jedoch auch andere Verbindungsmöglichkeiten zwischen dem Steuermodul und der dentalen oder medizinische Behandlungseinheit möglich.

Alternativ ist das Steuermodul integral und/ oder unlösbar mit der dentalen oder medizinischen Behandlungseinheit und/ oder der Schlauchleitung für das gasförmige Antriebsmedium und/ oder dem dentalen oder medizinischen, pneumatisch angetriebenen Behandlungswerkzeug ausgebildet. Dies ist insbesondere dann der Fall, wenn das Steuermodul bei der Herstellung der dentalen oder medizinischen Behandlungseinheit und/ oder des dentalen oder medizinischen, pneumatisch angetriebenen Behandlungswerkzeugs an der Schlauchleitung verbaut wird, i.e. wenn das Steuermodul kein Nachrüst-Steuermodul oder Umrüst-Steuermodul ist.

Das Steuermodul, insbesondere das Nachrüst-Steuermodul oder Umrüst-Steuermodul, weist vorzugsweise ein Gehäuse auf, das in vorteilhafter Weise den Anschluss an die dentale oder medizinische Behandlungseinheit erleichtert. Das Gehäuse bildet insbesondere auch eine Aufnahme und einen Schutz für das Stellelement, den Funkempfänger, ein im Steuermodul angeordnetes Schlauchstück und gegebenenfalls weitere im Folgenden genannte Komponenten des Steuermoduls.

Das Steuermodul, insbesondere das Nachrüst-Steuermodul oder Umrüst-Steuermodul, weist vorzugsweise auf: eine Aufnahmeöffnung, die mit der Ausgangsöffnung der dentalen oder medizinischen Behandlungseinheit verbindbar und/ oder an eine Schnittstelle der Schlauchleitung für das Antriebsmedium anschließbar ist; eine Abgabeöffnung, die mit der Einlassöffnung der dentalen oder medizinischen Behandlungseinheit verbindbar und/ oder an die Schnittstelle der Schlauchleitung für das Antriebsmedium anschließbar ist; und ein die Aufnahmeöffnung mit der Abgabeöffnung verbindendes Schlauchstück für das gasförmige Antriebsmedium. Das gasförmige Antriebsmedium kann somit aus der dentalen oder medizinischen Behandlungseinheit und/ oder einem ersten Abschnitt der Schlauchleitung für das Antriebsmedium über die Ausgangsöffnung und/ oder Schnittstelle und die Aufnahmeöffnung in das Steuermodul und durch das Schlauchstück des Steuermoduls, die Abgabeöffnung und die Einlassöffnung und/ oder Schnittstelle wiederum in die dentale oder medizinische Behandlungseinheit und/ oder einen zweiten Abschnitt der Schlauchleitung für das Antriebsmedium und weiter in das pneumatisch angetriebene Behandlungswerkzeug fließen. Der erste und zweite Abschnitt der Schlauchleitung für das Antriebsmedium bilden vorzugsweise gemeinsam die Schlauchleitung für das Antriebsmedium und sind insbesondere durch die Schnittstelle voneinander getrennt, wobei diese Schnittstelle zum Anschluss der pneumatischen Fußsteuerung (so wie aus dem Stand der Technik bekannt) oder erfindungsgemäß des Steuermoduls ausgebildet ist. Die Schnittstelle umfasst somit insbesondere die Ausgangsöffnung und die Einlassöffnung der dentalen oder medizinischen Behandlungseinheit, wobei die Ausgangsöffnung und die Einlassöffnung vorzugsweise durch Öffnungen der ersten und zweiten Abschnitte der Schlauchleitung gebildet sind. Das Schlauchstück des Steuermoduls ist dazu vorgesehen, die Ausgangsöffnung und die Einlassöffnung miteinander zu verbinden.

Das Stellelement des Steuermoduls weist insbesondere ein Ventil auf, wodurch in vorteilhafter Wiese eine besonders zuverlässige Steuerung des Volumenstroms des Antriebsmediums erzielt wird. Das Ventil ist zum Beispiel als Stetigventil, Proportionalventil, Steuerventil, Magnetventil oder Quetschventil ausgebildet.

Das Steuermodul weist vorzugsweise eine elektrische Energiequelle zur Energieversorgung des Steuermoduls, insbesondere des Stellelements und/ oder des Funkempfängers auf. Die Energiequelle umfasst vorzugsweise eine Batterie oder einen mehrmals aufladbaren Akkumulator, wodurch in vorteilhafter Weise auch der Volumenstrom des Antriebsmediums einer ausschließlich pneumatisch betriebenen dentalen oder medizinischen Behandlungseinheit, die keine elektrische Energiequelle oder keinen Anschluss an eine elektrische Energiequelle aufweist, mit dem Steuermodul gesteuert werden kann.

Die Energiequelle kann alternativ oder zusätzlich auch durch einen elektrodynamischen Wandler (Generator) gebildet sein, der in dem Steuermodul angeordnet und mit Druckgas versorgbar ist, so dass die elektrische Versorgung des Steuermoduls in vorteilhafter Weise durch in dem Steuermodul erzeugte elektrische Energie erfolgt. Der elektrodynamische Wandler ist insbesondere durch das Antriebsmedium antreibbar, das dem pneumatisch angetriebenen Behandlungswerkzeug zuleitbar ist oder zugeleitet wird. Besonders bevorzugt wird der elektrodynamische Wandler über eine separate Leitung von derselben Medienquelle wie das pneumatisch angetriebene Behandlungswerkzeug versorgt, so dass die Versorgung des Generators unabhängig von der Versorgung des pneumatisch angetriebenen Behandlungswerkzeugs ist.

Alternativ ist es natürlich auch möglich das Steuermodul mit elektrischer Energie aus einer externen elektrischen Energiequelle zu versorgen. Die externe elektrische Energiequelle ist zum Beispiel in der dentalen oder medizinischen Behandlungseinheit angeordnet oder durch einen Anschluss an ein externes Stromnetz gebildet.

Vorzugsweise umfasst das Steuermodul einen Mikrocontroller zum Steuern des Stellelements auf Basis des drahtlos übertragenen und empfangenen Funk-Stellsignals. Der Mikrocontroller ist insbesondere auch dazu ausgebildet, das empfangene Funk-Stellsignal zu verarbeiten.

Vorzugsweise ist der Funkempfänger des Steuermoduls mit einer Antenne zum Empfangen des drahtlos übertragenen Funk-Stellsignals der Funk-Fußsteuerung verbunden, wobei die Antenne insbesondere als Teil des Funkempfängers oder zumindest teilweise durch ein Gehäuse des Steuermoduls gebildet ist, wodurch in vorteilhafter Weise ein besserer Empfang des Funk-Stellsignals bei gleichzeitiger Reduktion der Größe des Funkempfängers ermöglicht wird.

Vorzugsweise ist der Funkempfänger ausgebildet, zumindest ein zusätzliches, drahtlos übertragenes Funk-Stellsignal der Funk-Fußsteuerung zu empfangen, so dass auf Basis des zusätzlichen drahtlos übertragenen und empfangenen Funk-Stellsignals ein zusätzlicher Betriebsparameter des dentalen oder medizinischen, pneumatisch angetriebenen Behandlungswerkzeugs stellbar oder steuerbar ist. Vorzugsweise verarbeitet der Mikrocontroller des Steuermoduls dieses zusätzliche, drahtlos übertragene Funk-Stellsignal. Vorzugsweise ist der Mikrocontroller mit zusätzlichen Stellelementen verbunden, die auf Basis des zusätzlichen drahtlos übertragenen und empfangenen Funk-Stellsignals durch den Mikrocontroller gestellt oder gesteuert werden. Alternativ oder zusätzlich ist der Mikrocontroller ausgebildet, das zusätzliche, drahtlos übertragene Funk-Stellsignal an eine zentrale Steuereinheit des pneumatisch angetriebenen Behandlungswerkzeugs oder der dentalen oder medizinischen Behandlungseinheit weiterzuleiten.

Vorzugsweise umfasst der zusätzliche Betriebsparameter zumindest einen der folgenden Parameter: eine Drehrichtung des Behandlungswerkzeugs, insbesondere einer Werkzeughalterung oder einer Antriebsvorrichtung des Behandlungswerkzeugs; einen Lichtabgabewert einer Beleuchtungsquelle des Behandlungswerkzeugs, zum Beispiel das Ein-/Ausschalten der Beleuchtungsquelle, die abgegebene Lichtintensität der Beleuchtungsquelle oder das Umschalten zwischen verschiedenen Beleuchtungsquellen; einen Kühlmittelabgabewert eines durch das pneumatische Behandlungswerkzeug abgegebenen Kühlmittels, zum Beispiel eines Kühlmittelsprays, insbesondere der Volumenstrom oder die mengenmäßige Zusammensetzung unterschiedlicher Komponenten eines Kühlmittels, zum Beispiel von Wasser und Luft; einen Betriebsmodus des Behandlungswerkzeugs, insbesondere die Auswahl unterschiedlicher Betriebsprogramme.

Vorzugsweise umfasst das Steuermodul, insbesondere das Nachrüst-Steuermodul oder Umrüst-Steuermodul, zumindest ein Kupplungselement zur, vorzugsweise lösbaren Verbindung mit der Schlauchleitung für das gasförmige Antriebsmedium und/ oder der Schnittstelle und/ oder dem pneumatisch angetriebenen Behandlungswerkzeug und/ oder der dentalen oder medizinischen Behandlungseinheit. Das zumindest eine Kupplungselement umfasst zum Beispiel ein Kupplungsrohr, ein Steckelement, ein Steckrohr, einen Kupplungsfortsatz, ein Formschlusselement, z.B. einen Federarm, eine Kupplungsaufnahme. Das zumindest eine Kupplungselement ist insbesondere zur Verbindung des Schlauchstücks des Steuermoduls mit der Schlauchleitung für das gasförmige Antriebsmedium vorgesehen oder fixiert diese Verbindung.

Vorzugsweise ist das zumindest eine Kupplungselement an der Aufnahmeöffnung und/ oder an der Abgabeöffnung des Steuermoduls angeordnet oder das zumindest eine Kupplungselement weist die Aufnahmeöffnung und/ oder die Abgabeöffnung auf.

Alternativ oder zusätzlich weist die dentale oder medizinische Behandlungseinheit und/ oder die Schlauchleitung für das gasförmige Antriebsmedium und/ oder die Schnittstelle zumindest ein Kupplungsstück auf, insbesondere an der Ausgangsöffnung und/ oder der Einlassöffnung. Vorzugsweise sind das zumindest eine Kupplungselement und das zumindest eine Kupplungsstück miteinander (lösbar) verbindbar, um insbesondere eine dichte Verbindung des Schlauchstücks des Steuermoduls mit der Schlauchleitung für das gasförmige Antriebsmedium zu gewährleisten.

Die drahtlose Übertragung des Funk-Stellsignals kann durch unterschiedliche bekannte Technologien implementiert sein, zum Beispiel mittels Bluetooth, Infrarot oder WiFi.

Wie im Vorstehenden bereits beschrieben ist das Steuermodul vorzugsweise so ausgebildet, dass es an einer Schnittstelle der Schlauchleitung für das gasförmige Antriebsmedium befestigbar ist, die für die Verbindung mit einer pneumatischen Fußsteuerung vorgesehen ist. Ein derartiges Steuermodul ist somit insbesondere als Umrüst-Einheit oder Nachrüst-Einheit ausgebildet, die vorzugsweise nachträglich an der Schlauchleitung für das Antriebsmedium befestigbar ist, um eine pneumatische Fußsteuerung durch eine drahtlose und schlauchlose Funk-Fußsteuerung zu ersetzen.

Vorzugsweise ist die Schnittstelle der Schlauchleitung für das gasförmige Antriebsmedium an einer dentalen oder medizinischen Behandlungseinheit vorgesehen, mit der das pneumatisch angetriebene Behandlungswerkzeug verbindbar oder verbunden ist. Die Schnittstelle weist eine mit einer Quelle für das gasförmige Antriebsmedium, insbesondere Druckluft verbundene Ausgangsöffnung und eine mit dem pneumatisch angetriebenen Behandlungswerkzeug verbundene Einlassöffnung auf, wobei die Ausgangsöffnung und die Einlassöffnung insbesondere an der Schlauchleitung für das gasförmige Antriebsmedium bzw. an (ersten und zweiten) Abschnitten dieser Schlauchleitung vorgesehen sind. Das Steuermodul weist eine mit der Ausgangsöffnung der Schnittstelle verbindbare Aufnahmeöffnung, eine mit der Einlassöffnung der Schnittstelle verbindbare Abgabeöffnung und ein die Aufnahmeöffnung mit der Abgabeöffnung verbindendes Schlauchstück für das gasförmige Antriebsmedium auf, so dass das gasförmige Antriebsmedium aus der dentalen oder medizinischen Behandlungseinheit über die Ausgangsöffnung und die Aufnahmeöffnung in das Steuermodul und durch das Schlauchstück, die Abgabeöffnung und die Einlassöffnung wiederum in die dentale oder medizinische Behandlungseinheit und in das pneumatisch angetriebene Behandlungswerkzeug fließen kann. Das Stellelement zum Stellen des Volumenstroms des gasförmigen Antriebsmediums ist an dem die Aufnahmeöffnung mit der Abgabeöffnung verbindenden Schlauchstück des Steuermoduls vorgesehen.

Vorzugsweise umfasst die dentale oder medizinische Behandlungseinheit zusätzlich zu dem pneumatisch angetriebenen Behandlungswerkzeug zumindest ein elektrisch angetriebenes Behandlungswerkzeug, das durch eine zentrale oder zusätzliche Steuereinheit der dentalen oder medizinischen Behandlungseinheit gesteuert ist. Besonders bevorzugt ist die zentrale oder zusätzliche Steuereinheit mit dem Mikrocontroller des Steuermoduls kommunikativ verbunden, wobei der Mikrocontroller des Steuermoduls ausgebildet ist, das vom Funkempfänger des Steuermoduls empfangene drahtlos übertragene Funk-Stellsignal einer Funk-Fußsteuerung an die zentrale oder zusätzliche Steuereinheit (drahtlos oder drahtgebunden) weiterzuleiten und wobei die zentrale oder zusätzliche Steuereinheit ausgebildet ist, das weitergeleitete Funk-Stellsignal zu empfangen und zu verarbeiten, um das zumindest eine elektrisch angetriebene Behandlungswerkzeug zu steuern. Damit ist in vorteilhafter Weise mit dem Steuermodul zusätzlich auch der Betrieb eines elektrisch angetriebenen Behandlungswerkzeugs steuerbar, insbesondere dessen Drehzahl oder gegebenenfalls weitere Parameter wie zum Beispiel die Drehrichtung, Lichtabgabe, Spraymengenabgabe und/ oder die im Vorstehenden beschriebenen zusätzlichen Betriebsparameter.

Gemäß einem besonders bevorzugten Ausführungsbeispiel ist ein Nachrüst-Steuermodul oder Umrüst-Steuermodul zur insbesondere drahtlosen und/ oder schlauchlosen und/ oder funkgesteuerten Steuerung eines Volumenstroms eines gasförmigen Antriebsmediums eines dentalen oder medizinischen, pneumatisch angetriebenen Behandlungswerkzeugs mit einer Fußsteuerung vorgesehen, wobei das pneumatisch angetriebene Behandlungswerkzeug mit einer dentalen oder medizinischen Behandlungseinheit verbindbar oder verbunden ist, wobei die dentale oder medizinische Behandlungseinheit eine Ausgangsöffnung, die mit einer Quelle für das gasförmige Antriebsmedium verbunden ist, und eine Einlassöffnung aufweist, die mit dem pneumatisch angetriebenen Behandlungswerkzeug verbunden ist, wobei das Steuermodul eine mit der Ausgangsöffnung der dentalen oder medizinischen Behandlungseinheit verbindbare Aufnahmeöffnung, eine mit der Einlassöffnung der dentalen oder medizinischen Behandlungseinheit verbindbare Abgabeöffnung und ein die Aufnahmeöffnung mit der Abgabeöffnung verbindendes Schlauchstück für das gasförmige Antriebsmedium umfasst, so dass das gasförmige Antriebsmedium aus der dentalen oder medizinischen Behandlungseinheit über die Ausgangsöffnung und die Aufnahmeöffnung in das Steuermodul und durch das Schlauchstück, die Abgabeöffnung und die Einlassöffnung wiederum in die dentale oder medizinische Behandlungseinheit und in das pneumatisch angetriebene Behandlungswerkzeug fließt, wobei an dem Schlauchstück des Steuermoduls ein Stellelement angeordnet ist, das ausgebildet ist, den Volumenstrom des durch das Schlauchstück fließenden gasförmigen Antriebsmediums zu stellen, und wobei das Steuermodul einen Funkempfänger aufweist, der ausgebildet ist, ein drahtlos übertragenes Funk-Stellsignal einer Funk-Fußsteuerung zu empfangen, so dass das Stellelement auf Basis des drahtlos übertragenen und empfangenen Funk-Stellsignals den Volumenstrom des durch das Schlauchstück fließenden gasförmigen Antriebsmediums stellt.

Mit diesen Nachrüst-Steuermodulen oder Umrüst-Steuermodulen ist es in besonders einfacher Weise möglich, eine vorhandene pneumatische Fußsteuerung durch ein erfindungsgemäßes Steuermodul zu ersetzen und damit eine drahtlose und/ oder schlauchlose und/ oder funkgesteuerte Steuerung des Volumenstroms des gasförmigen Antriebsmediums bzw. der Drehzahl oder Leistung des Behandlungswerkzeugs zu ermöglichen.

Vorzugsweise ist ein Set für ein dentales oder medizinisches, pneumatisch angetriebenes Behandlungswerkzeug oder eine dentale oder medizinische Behandlungseinheit vorgesehen, die ein im Vorstehenden beschriebenes Steuermodul, insbesondere ein Nachrüst-Steuermodul oder Umrüst-Steuermodul, und eine Funk-Fußsteuerung aufweist, die zur (drahtlosen und schlauchlosen) Übertragung zumindest eines Funk-Stellsignals an das Steuermodul zum Stellen des Volumenstroms des durch die Schlauchleitung, insbesondere das Schlauchstück des Steuermoduls fließenden gasförmigen Antriebsmediums ausgebildet ist.

Vorzugsweise umfasst eine mit dem Steuermodul verwendbare oder koppelbare Funk-Fußsteuerung, insbesondere die Funk-Fußsteuerung des Sets für ein dentales oder medizinisches, pneumatisch angetriebenes Behandlungswerkzeug: einen Funksender zum Senden des drahtlos übertragenen Funk-Stellsignals zum Stellen des Volumenstroms des gasförmigen Antriebsmediums (durch das Stellelement des Steuermoduls) an den Funkempfänger des Steuermoduls; eine Energiequelle zur Energieversorgung der Funk-Fußsteuerung, insbesondere des Funksenders und gegebenenfalls weiterer elektrische Energie benötigender Komponenten der Funk-Fußsteuerung, insbesondere eines Mikrocontrollers; zumindest eine Benutzerschnittstelle, insbesondere ein dreh- oder schwenkbares Pedal, über die der Anwender Stellbefehle zum Stellen des Volumenstroms des gasförmigen Antriebsmediums erzeugt; und einen operativ mit der Benutzerschnittstelle und mit dem Funksender verbundenen Mikrocontroller, der ausgebildet ist, die über die Benutzerschnittstelle erzeugten Stellbefehle des Anwenders zu empfangen, vorzugsweise zu verarbeiten und auf Basis der empfangenen (und verarbeiteten) Stellbefehle den Funksender zu steuern, so dass der Funksender das Funk-Stellsignal zum Stellen des Volumenstroms des gasförmigen Antriebsmediums drahtlos an den Funkempfänger des Steuermoduls überträgt.

Vorzugsweise umfasst die Funk-Fußsteuerung zumindest eine zusätzliche Benutzerschnittstelle, über die der Anwender einen zusätzlichen Stellbefehl zum Stellen eines zusätzlichen Betriebsparameters für das dentale oder medizinische, pneumatisch angetriebene Behandlungswerkzeug erzeugt, so wie dies im Vorstehenden bereits beschrieben ist. Vorzugsweise ist der mit dem Funksender verbundene Mikrocontroller ausgebildet, den über die zusätzliche Benutzerschnittstelle erzeugten zusätzlichen Stellbefehl des Anwenders zu empfangen und gegebenenfalls zu verarbeiten und auf Basis des zusätzlichen empfangenen (und verarbeiteten) Stellbefehls den Funksender zu steuern, so dass der Funksender das zusätzliche Funk-Stellsignal zum Stellen des zusätzlichen Betriebsparameters drahtlos an den Funkempfänger überträgt. Die zusätzliche Benutzerschnittstelle ist zum Beispiel als dreh- oder schwenkbares Pedal, Schieber oder Taster ausgebildet.

Vorzugsweise ist eine dentale oder medizinische Behandlungseinheit vorgesehen, die mit einem dentalen oder medizinischen, pneumatisch angetriebenen Behandlungswerkzeug verbunden oder verbindbar ist, wobei die dentale oder medizinische Behandlungseinheit ein im Vorstehenden beschriebenes Steuermodul zur Steuerung eines Volumenstroms eines gasförmigen Antriebsmediums eines dentalen oder medizinischen, pneumatisch angetriebenen Behandlungswerkzeugs oder ein im Vorstehenden beschriebenes Set für ein dentales oder medizinisches, pneumatisch angetriebenes Behandlungswerkzeug aufweist. Das Steuermodul ist insbesondere als Nachrüst-Steuermodul oder Umrüst-Steuermodul ausgebildet.

Die dentale oder medizinische Behandlungseinheit umfasst zum Beispiel einen Patientenstuhl, eine Ablage für zumindest ein dentales oder medizinisches, pneumatisch angetriebenes Behandlungswerkzeug, einen zentralen Mikrocontroller zur Steuerung der dentalen oder medizinischen Behandlungseinheit und eine Quelle für das gasförmige Antriebsmedium oder einen Anschluss an eine Quelle für das gasförmige Antriebsmedium. Alternativ umfasst die dentale oder medizinische Behandlungseinheit zum Beispiel: ein Tischsteuergerät; eine mobile und/ oder autarke Behandlungseinheit, wie sie insbesondere in militärischen Einrichtungen oder bei der Behandlung bettlägerig Patienten verwendet wird.

Die dentale oder medizinische Behandlungseinheit umfasst insbesondere Behandlungseinheiten, die ausschließlich pneumatisch betriebene Behandlungswerkzeuge aufweisen, oder Behandlungseinheiten, deren Behandlungswerkzeuge für die dentale Restauration und/ oder Prothetik ausschließlich pneumatisch betriebene Behandlungswerkzeug sind (wobei zusätzliche Behandlungswerkzeuge, die nicht für die dentale Restauration und/ oder Prothetik vorgesehen sind, zum Beispiel Behandlungswerkzeuge für implantologische, endodontische oder parodontisch Behandlungen auch andere als pneumatische Antriebe aufweisen können, insbesondere elektrische Antriebe).

Ein Verfahren zum Umrüsten oder Nachrüsten eines dentalen oder medizinischen, pneumatisch angetriebenen Behandlungswerkzeugs umfasst, dass ein im Vorstehenden beschriebenes Steuermodul, insbesondere ein Nachrüst-Steuermodul oder ein Umrüst-Steuermodul, zur Steuerung eines Volumenstroms eines gasförmigen Antriebsmediums an einer Schnittstelle der Schlauchleitung für das Antriebsmedium, die für die Verbindung mit einer pneumatischen Fußsteuerung vorgesehen ist, befestigt wird. Gegebenenfalls wird/ werden vorher die pneumatische Fußsteuerung und insbesondere auch die Schlauchleitungen von der pneumatischen Fußsteuerung zum dentalen oder medizinischen, pneumatisch angetriebenen Behandlungswerkzeug oder zur dentalen oder medizinischen Behandlungseinheit entfernt. Vorzugsweise wird auch eine mit dem Steuermodul verwendbare oder koppelbare Funk-Fußsteuerung zur Verfügung gestellt. Besonders bevorzugt wird in einem weiteren Schritt der Funksender der Funk-Fußsteuerung mit dem Funkempfänger des Steuermoduls gekoppelt (gepaired).

Die Erfindung wird nachfolgend anhand bevorzugter Ausführungsbeispiele und Bezug nehmend auf die beigefügten Zeichnungen erläutert.
Figur 1 zeigt in einer schematischen Ansicht die aus dem Stand der Technik bekannte Steuerung eines Volumenstroms eines gasförmigen Antriebsmediums eines dentalen oder medizinischen, pneumatisch angetriebenen Behandlungswerkzeugs mit einer pneumatischen Fußsteuerung.
Figur 2 zeigt in einer schematischen Ansicht eine drahtlose und schlauchlose Steuerung eines Volumenstroms eines gasförmigen Antriebsmediums eines dentalen oder medizinischen, pneumatisch angetriebenen Behandlungswerkzeugs mit einer Funk-Fußsteuerung.
Figur 3 zeigt in einer schematischen Ansicht eine Funk-Fußsteuerung zur drahtlosen und schlauchlosen Steuerung eines Volumenstroms eines gasförmigen Antriebsmediums eines dentalen oder medizinischen, pneumatisch angetriebenen Behandlungswerkzeugs.
Figur 4 zeigt in einer schematischen Ansicht ein Steuermodul zur drahtlosen und schlauchlosen Steuerung eines Volumenstroms eines gasförmigen Antriebsmediums eines dentalen oder medizinischen, pneumatisch angetriebenen Behandlungswerkzeugs.
Figur 5 zeigt in einer schematischen Ansicht eine Verbindungsseite eines Steuermoduls, insbesondere eines Nachrüst-Steuermoduls oder eines Umrüst-Steuermoduls, zur drahtlosen und schlauchlosen Steuerung eines Volumenstroms eines gasförmigen Antriebsmediums eines dentalen oder medizinischen, pneumatisch angetriebenen Behandlungswerkzeugs.

Die in der Figur 2 dargestellte dentale oder medizinische Behandlungseinheit 1 umfasst ein Steuermodul 10, 10A zur Steuerung eines Volumenstroms eines gasförmigen Antriebsmediums eines dentalen oder medizinischen, pneumatisch angetriebenen Behandlungswerkzeugs 5. Das gasförmige Antriebsmedium fließt durch eine Schlauchleitung 4 zu dem pneumatisch angetriebenen Behandlungswerkzeug 5, wobei sich die Schlauchleitung 4 vorzugsweise durch die dentale oder medizinische Behandlungseinheit 1 erstreckt und mit einer Quelle für das gasförmige Antriebsmedium, insbesondere Druckluft verbunden ist. Die Quelle für das gasförmige Antriebsmedium umfasst insbesondere einen Kompressor.

Das Steuermodul 10, 10A umfasst ein Stellelement 9, insbesondere ein Ventil 9A (siehe Figur 4), das ausgebildet ist, den Volumenstrom des zum dentalen oder medizinischen, pneumatisch angetriebenen Behandlungswerkzeugs 5 fließenden gasförmigen Antriebsmediums zu stellen, um damit die Drehzahl und/ oder Leistung des dentalen oder medizinischen, pneumatisch angetriebenen Behandlungswerkzeugs 5 zu steuern. Das Steuermodul 10, 10A umfasst des Weiteren einen Funkempfänger 8, der ausgebildet ist, ein drahtlos übertragenes Funk-Stellsignal einer Funk-Fußsteuerung 3 zu empfangen, wobei der Funkempfänger 8 und das Stellelement 9 kommunikativ verbunden sind, so dass das Stellelement 9 auf Basis des drahtlos übertragenen und empfangenen Funk-Stellsignals den Volumenstrom des zum dentalen oder medizinischen, pneumatisch angetriebenen Behandlungswerkzeugs 5 fließenden gasförmigen Antriebsmediums stellt.

Das Steuermodul 10, 10A kann integral und/ oder unlösbar mit der Schlauchleitung 4 für das gasförmige Antriebsmedium ausgebildet sein und insbesondere in oder an der dentalen oder medizinischen Behandlungseinheit 1 verbaut sein.

Alternativ ist das Steuermodul 10, 10A an einer Schnittstelle 14 der Schlauchleitung 4 für das Antriebsmedium angeordnet, die insbesondere ursprünglich für die Verbindung mit einer pneumatischen Fußsteuerung 103 vorgesehen ist. Dies wird aus dem Vergleich der Figuren 1 und 2 ersichtlich: die Schnittstelle 14 weist gemäß dem Ausführungsbeispiel nach Figur 1 die Auslassöffnung 106 und die Einlassöffnung 107 auf. Nach dem Entfernen der pneumatischen Fußsteuerung 103 und der Schlauchleitungen 101, 102 kann das Steuermodul 10, 10A an der Schnittstelle 14 befestigt werden. Die Schnittstelle 14 ist insbesondere an der dentalen oder medizinischen Behandlungseinheit 1 vorgesehen.

Zum Befestigen und Verbinden des Steuermoduls 10, das insbesondere als Nachrüst-Steuermodul oder Umrüst-Steuermodul 10A ausgebildet ist (siehe Figur 5), an der Schnittstelle 14 weist das Steuermodul 10, 10A eines oder mehrere, zum Beispiel zwei, Kupplungselemente 13 auf. Das zumindest eine Kupplungselement 13 ist ausgebildet, mit der dentalen oder medizinischen Behandlungseinheit 1, insbesondere mit der oder an die Schnittstelle 14, verbunden oder gekoppelt zu werden, so dass das Steuermodul 10 an der Schnittstelle 14 angeordnet ist. Durch das Befestigen und Verbinden des Steuermoduls 10 an der Schnittstelle 14 ist eine fluidleitende Verbindung zwischen dem Steuermodul 10 und der Schlauchleitung 4 für das gasförmige Antriebsmedium herstellbar, bei welcher insbesondere eine Aufnahmeöffnung 16 des Steuermoduls 10 mit der Ausgangsöffnung 106 der Schnittstelle 14 und eine Abgabeöffnung 17 des Steuermoduls 10 mit der Einlassöffnung 107 der Schnittstelle 14 fluidleitend verbunden ist. Die zwei Kupplungselemente 13 umfassen hierbei zum Beispiel ein Rohrstück, das in die Ausgangsöffnung 106 und in die Einlassöffnung 107 einsteckbar ist. An den Kupplungselementen 13 sind vorzugsweise Dichtelemente vorgesehen, welche ein Austreten von Antriebsfluid an der Schnittstelle 14 verhindern.

Die Aufnahmeöffnung 16 und die Abgabeöffnung 17 des Steuermoduls 10, vorzugsweise auch das zumindest eine Kupplungselement 13, sind an einer Außenwand und/ oder Seitenwand, zum Beispiel einer Rückwand eines Gehäuses 23 des Steuermoduls 10 angeordnet (siehe Figur 5).

Die Aufnahmeöffnung 16 und die Abgabeöffnung 17 sind innerhalb des Steuermoduls 10 durch ein Schlauchleitungsstück 15 (Figur 4) miteinander verbunden. An diesem Schlauchleitungsstück 15 ist das Stellelement 9 angeordnet, um den Volumenstrom des durch die Schlauchleitung 4, 15 zu dem dentalen oder medizinischen, pneumatisch angetriebenen Behandlungswerkzeugs 5 fließenden gasförmigen Antriebsmediums zu stellen, um damit die Drehzahl und/ oder Leistung des dentalen oder medizinischen, pneumatisch angetriebenen Behandlungswerkzeugs 5 zu steuern. Das Schlauchleitungsstück 15 ist durch den Anschluss des Steuermoduls 10 mit der Schlauchleitung 4 fluidleitend verbunden oder in die Schlauchleitung 4 (als Teil der Schlauchleitung 4) integriert.

In der Figur 4 ist des Weiteren dargestellt, dass das Steuermodul 10, 10A eine elektrische Energiequelle 11 zur Energieversorgung des Steuermoduls 10, 10A, insbesondere des Stellelements 9 und/ oder des Funkempfängers 8 aufweist. Die elektrische Energiequelle 11 umfasst insbesondere eine Batterie oder einen mehrmals aufladbaren Akkumulator.

In dem Steuermodul 10, 10A ist auch ein Mikrocontroller 12 zum Steuern des Stellelements 9 auf Basis des drahtlos übertragenen und empfangenen Funk-Stellsignals enthalten. Vorzugsweise verarbeitet der Mikrocontroller 12 das empfangene Funk-Stellsignal.

Der Funkempfänger 8 weist eine Antenne 8A zum Empfangen des drahtlos übertragenen Funk-Stellsignals der Funk-Fußsteuerung 3 auf.

Die in der Figur 3 dargestellte Funk-Fußsteuerung 3 umfasst einen Funksender 18 zum Senden des drahtlos übertragenen Funk-Stellsignals zum Stellen des Volumenstroms des gasförmigen Antriebsmediums an den Funkempfänger 8 des Steuermoduls 10, 10A. Der Funksender 18 sendet insbesondere mittels Bluetooth. In der Funk-Fußsteuerung 3 ist des Weiteren eine Energiequelle 19 zur Energieversorgung der Funk-Fußsteuerung 3, insbesondere des Funksenders 18 vorgesehen.

Über zumindest eine Benutzerschnittstelle 20 der Funk-Fußsteuerung 3 erzeugt der Anwender Stellbefehle zum Stellen des Volumenstroms des gasförmigen Antriebsmediums und damit der Drehzahl oder der Leistung des dentalen oder medizinischen, pneumatisch angetriebenen Behandlungswerkzeugs 5. Die Benutzerschnittstelle 20 ist zum Beispiel als Pedal oder (relativ zu einem Gehäuse der Funk-Fußsteuerung 3) verschwenkbares, verschiebbares oder drehbares Bedienelement ausgebildet.

Optional weist die Funk-Fußsteuerung 3 zumindest eine zusätzliche Benutzerschnittstelle 22 auf, über die der Anwender einen zusätzlichen Stellbefehl zum Stellen eines zusätzlichen Betriebsparameters für das dentale oder medizinische, pneumatisch angetriebene Behandlungswerkzeug 5 erzeugt. Die Benutzerschnittstelle 20 ist zum Beispiel als Pedal oder (relativ zu einem Gehäuse der Funk-Fußsteuerung 3) verschwenkbares, verschiebbares oder drehbares Bedienelement ausgebildet.

Ein in der Funk-Fußsteuerung 3 angeordneter Mikrocontroller 21 ist operativ mit der Benutzerschnittstelle 20, gegebenenfalls mit der zusätzliche Benutzerschnittstelle 22 und mit dem Funksender 18 verbunden. Der Mikrocontroller 21 ist ausgebildet, die über die Benutzerschnittstelle 20 und gegebenenfalls über die zusätzliche Benutzerschnittstelle 22 erzeugten Stellbefehle des Anwenders zu empfangen und auf Basis der empfangenen Stellbefehle den Funksender 18 derart zu steuern, dass der Funksender 18 das Funk-Stellsignal zum Stellen des Volumenstroms des gasförmigen Antriebsmediums drahtlos und gegebenenfalls das zusätzliche Funk-Stellsignal überträgt.

Die beschriebenen oder dargestellten Ausführungsbeispiele dienen insbesondere der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines der anderen Ausführungsbeispiele kombinierbar.

## Patentansprüche

1. Steuermodul (10; 10A) zur Steuerung eines Volumenstroms eines gasförmigen Antriebsmediums eines dentalen oder medizinischen, pneumatisch angetriebenen Behandlungswerkzeugs (5), wobei das gasförmige Antriebsmedium durch eine Schlauchleitung (4; 15) zu dem pneumatisch angetriebenen Behandlungswerkzeug (5) fließt und wobei an der Schlauchleitung (4; 15) ein Stellelement (9) vorgesehen ist, das ausgebildet ist, den Volumenstrom des durch die Schlauchleitung (4; 15) fließenden gasförmigen Antriebsmediums zu stellen, um damit die Drehzahl und/ oder Leistung des dentalen oder medizinischen, pneumatisch angetriebenen Behandlungswerkzeugs (5) zu steuern, wobei das Steuermodul (10; 10A) das Stellelement (9) umfasst, **dadurch gekennzeichnet, dass**
das Steuermodul (10; 10A) des Weiteren einen Funkempfänger (8) aufweist, der ausgebildet ist, ein drahtlos übertragenes Funk-Stellsignal einer Funk-Fußsteuerung (3) zu empfangen, wobei der Funkempfänger (8) und das Stellelement (9) kommunikativ verbunden sind, so dass das Stellelement (9) auf Basis des drahtlos übertragenen und empfangenen Funk-Stellsignals den Volumenstrom des zu dem dentalen oder medizinischen, pneumatisch angetriebenen Behandlungswerkzeug (5) fließenden gasförmigen Antriebsmediums stellt.

2. Steuermodul (10; 10A) nach Anspruch 1, **dadurch gekennzeichnet, dass**
das Stellelement (9) ein Ventil (9A) aufweist.

3. Steuermodul (10; 10A) nach Anspruch 1 oder 2, **gekennzeichnet durch** eine elektrische Energiequelle (11) zur Energieversorgung des Steuermoduls (10; 10A), insbesondere des Stellelements (9) und/ oder des Funkempfängers (8).

4. Steuermodul (10; 10A) nach einem der vorstehenden Ansprüche, **gekennzeichnet durch**
einen Mikrocontroller (12) zum Steuern des Stellelements (9) auf Basis des drahtlos übertragenen und empfangenen Funk-Stellsignals.

5. Steuermodul (10; 10A) nach einem der vorstehenden Ansprüche, **gekennzeichnet durch**
eine mit dem Funkempfänger (8) verbundene Antenne (8A) zum Empfangen des drahtlos übertragenen Funk-Stellsignals der Funk-Fußsteuerung (3), wobei die Antenne (8A) insbesondere als Teil des Funkempfängers (8) oder zumindest teilweise **durch** ein Gehäuse des Steuermoduls (10; 10A) gebildet ist.

6. Steuermodul (10; 10A) nach einem der vorstehenden Ansprüche, **dadurch gekennzeichnet, dass**
der Funkempfänger (8) ausgebildet ist, zumindest ein zusätzliches, drahtlos übertragenes Funk-Stellsignal der Funk-Fußsteuerung (3) zu empfangen, so dass auf Basis des zusätzlichen drahtlos übertragenen und empfangenen Funk-Stellsignals ein zusätzlicher Betriebsparameter des dentalen oder medizinischen, pneumatisch angetriebenen Behandlungswerkzeugs (5) stellbar ist.

7. Steuermodul (10; 10A) nach Anspruch 6, **dadurch gekennzeichnet, dass**
der zusätzliche Betriebsparameter zumindest einen der folgenden Parameter umfasst: eine Drehrichtung des Behandlungswerkzeugs (5); einen Lichtabgabewert einer Beleuchtungsquelle des Behandlungswerkzeugs (5); einen Kühlmittelabgabewert eines durch das Behandlungswerkzeug (5) abgegebenen Kühlmittels; einen Betriebsmodus des Behandlungswerkzeugs (5).

8. Steuermodul (10; 10A) nach einem der vorstehenden Ansprüche, **gekennzeichnet durch**
zumindest ein Kupplungselement (13) zur Verbindung des Steuermoduls (10; 10A) mit der Schlauchleitung (4) für das Antriebsmedium.

9. Steuermodul (10; 10A) nach einem der vorstehenden Ansprüche, **dadurch gekennzeichnet, dass**
das Steuermodul (10; 10A) an einer Schnittstelle (14) der Schlauchleitung (4) für das Antriebsmedium, die für die Verbindung mit einer pneumatischen Fußsteuerung (103) vorgesehen ist, befestigbar ist.

10. Steuermodul (10; 10A) nach Anspruch 9, **dadurch gekennzeichnet, dass**
das pneumatisch angetriebene Behandlungswerkzeug (5) mit einer dentalen oder medizinischen Behandlungseinheit (1) verbindbar oder verbunden ist, wobei die dentale oder medizinische Behandlungseinheit (1) die Schnittstelle (14) der Schlauchleitung (4) für das gasförmige Antriebsmedium zur einer pneumatischen Fußsteuerung (103) aufweist, wobei die Schnittstelle (14) eine mit einer Quelle für das gasförmige Antriebsmedium verbundene Ausgangsöffnung (106) und eine mit dem pneumatisch angetriebenen Behandlungswerkzeug (5) verbundene Einlassöffnung (107) aufweist, wobei das Steuermodul (10; 10A) eine mit der Ausgangsöffnung (106) der Schnittstelle (14) verbindbare Aufnahmeöffnung (16), eine mit der Einlassöffnung (107) der Schnittstelle (14) verbindbare Abgabeöffnung (17) und ein die Aufnahmeöffnung (16) mit der Abgabeöffnung (17) verbindendes Schlauchstück (15) für das gasförmige Antriebsmedium aufweist, so dass das gasförmige Antriebsmedium aus der dentalen oder medizinischen Behandlungseinheit (1) über die Ausgangsöffnung (106) und die Aufnahmeöffnung (16) in das Steuermodul (10; 10A) und durch das Schlauchstück (15), die Abgabeöffnung (17) und die Einlassöffnung (107) wiederum in die dentale oder medizinische Behandlungseinheit (1) und in das pneumatisch angetriebene Behandlungswerkzeug (5) fließen kann, wobei das Stellelement (9) an dem die Aufnahmeöffnung (16) mit der Abgabeöffnung (17) verbindenden Schlauchstück (15) des Steuermoduls (10; 10A) vorgesehen ist.

11. Set für eine dentales oder medizinisches, pneumatisch angetriebenes Behandlungswerkzeug (5), **gekennzeichnet durch**
ein Steuermodul (10; 10A) nach einem der vorstehenden Ansprüche und eine Funk-Fußsteuerung (3), die zur Übertragung zumindest eines Funk-Stellsignals an das Steuermodul (10; 10A) zum Stellen des Volumenstroms des gasförmigen Antriebsmediums ausgebildet ist.

12. Set nach Anspruch 11, **dadurch gekennzeichnet, dass**
die Funk-Fußsteuerung (3) umfasst: einen Funksender (18) zum Senden des drahtlos übertragenen Funk-Stellsignals zum Stellen des Volumenstroms des gasförmigen Antriebsmediums an den Funkempfänger (8) des Steuermoduls (10; 10A); eine Energiequelle (19) zur Energieversorgung der Funk-Fußsteuerung (3), insbesondere des Funksenders (18); zumindest eine Benutzerschnittstelle (20) über die der Anwender Stellbefehle zum Stellen des Volumenstroms des gasförmigen Antriebsmediums erzeugt; und einen operativ mit der Benutzerschnittstelle (20) und mit dem Funksender (18) verbundenen Mikrocontroller (21), der ausgebildet ist, die über die Benutzerschnittstelle (20) erzeugten Stellbefehle des Anwenders zu empfangen und auf Basis der empfangenen Stellbefehle den Funksender (18) zu steuern, so dass der Funksender (18) das Funk-Stellsignal zum Stellen des Volumenstroms des gasförmigen Antriebsmediums drahtlos überträgt.

13. Set nach Anspruch 12, **dadurch gekennzeichnet, dass**
die Funk-Fußsteuerung (3) zumindest eine zusätzliche Benutzerschnittstelle (22) aufweist, über die der Anwender einen zusätzlichen Stellbefehl zum Stellen eines zusätzlichen Betriebsparameters für das dentale oder medizinische, pneumatisch angetriebene Behandlungswerkzeug (5) erzeugt, wobei der mit dem Funksender (18) verbundene Mikrocontroller (21) ausgebildet ist, den über die zumindest eine zusätzliche Benutzerschnittstelle (22) erzeugten zusätzlichen Stellbefehl des Anwenders zu empfangen und auf Basis des zusätzlichen empfangenen Stellbefehls den Funksender (18) zu steuern, so dass der Funksender (18) das zusätzliche Funk-Stellsignal zum Stellen des zusätzlichen Betriebsparameters drahtlos überträgt.

14. Dentale oder medizinische Behandlungseinheit (1), die mit einem dentalen oder medizinischen, pneumatisch angetriebenen Behandlungswerkzeug (5) verbunden oder verbindbar ist, wobei die dentale oder medizinische Behandlungseinheit (1) **gekennzeichnet ist, durch**
ein Steuermodul (10; 10A) zur Steuerung eines Volumenstroms eines gasförmigen Antriebsmediums eines dentalen oder medizinischen, pneumatisch angetriebenen Behandlungswerkzeugs (5) nach einem der Ansprüche 1 - 10 oder **durch** ein Set für ein dentales oder medizinisches, pneumatisch angetriebenes Behandlungswerkzeug (5) nach einem der Ansprüche 11 - 13.

15. Verfahren zum Umrüsten oder Nachrüsten eines dentalen oder medizinischen, pneumatisch angetriebenen Behandlungswerkzeugs (5), **dadurch gekennzeichnet, dass**
ein Steuermodul (10; 10A) zur Steuerung eines Volumenstroms eines gasförmigen Antriebsmediums eines dentalen oder medizinischen, pneumatisch angetriebenen Behandlungswerkzeugs (5) gemäß einem der Ansprüche 1 - 10 an einer Schnittstelle (14) der Schlauchleitung (4) für das Antriebsmedium, die für die Verbindung mit einer pneumatischen Fußsteuerung (103) vorgesehen ist, befestigt wird.
